(19) Europäisches Patentamt
European Patent Office
Office européen des brevets

(11) EP 1 158 056 A1

(12) **EUROPEAN PATENT APPLICATION**

(43) Date of publication:
**28.11.2001 Bulletin 2001/48**

(51) Int Cl.[7]: **C12Q 1/68**

(21) Application number: **00201725.9**

(22) Date of filing: **15.05.2000**

(84) Designated Contracting States:
**AT BE CH CY DE DK ES FI FR GB GR IE IT LI LU MC NL PT SE**
Designated Extension States:
**AL LT LV MK RO SI**

(71) Applicant: **KEYGENE N.V.**
**6700 AE Wageningen (NL)**

(72) Inventors:
- **Van Eijk, Michael Josephus Theresia**
  **3993 ST Houten (NL)**
- **Peleman, Johan Dominicus**
  **6707 SG Wageningen (NL)**
- **De Ruiter-Bleeker, Maria Johanna**
  **6871 JM Renkum (NL)**

(74) Representative: **Jorritsma, Ruurd**
**Nederlandsch Octrooibureau**
**Scheveningseweg 82**
**P.O. Box 29720**
**2502 LS Den Haag (NL)**

(54) **Microsatellite-AFLP**

(57) The invention relates to the use of (the combination of) at least one RAMP-primer and at least one AFLP-primer in analysing a nucleic acid sequence, in particular in analysing a nucleic acid sequence for (the presence or absence of) polymorphisms/markers associated with microsatellites.

The invention also relates to the use of (the combination of) at least one RAMP-primer and at least one AFLP-primer in amplifying a nucleic acid sequence, and in particular a restriction fragment to which at least one adapter sequence, and preferably two adapter sequences, has/have been ligated, which may be part of a mixture of such adapter-ligated restriction fragments, and which preferably contains or is suspected to contain, a microsatellite.

The invention also relates to a method for analysing a nucleic acid sequence, said method comprising the steps of:

(a) restricting the starting nucleic acid with at least one restriction endonuclease to provide a mixture of restriction fragments;
(b) ligating the restriction fragments thus obtained to at least one adapter;
(c) amplifying the mixture of adapter-ligated restriction fragments thus obtained with at least one RAMP-primer and at least one AFLP-primer to provide a mixture of amplified restriction fragments;
and optionally comprising the further step of:
(d) detecting at least one of the amplified restriction fragments thus obtained.

EP 1 158 056 A1

## Description

The present invention relates to a method for identifying and/or analysing nucleic acid sequences.

In particular, the invention relates to a method for identifying and/or analysing nucleic acid sequences based upon the occurrance in said nucleic acid sequences of highly repetitive nucleotide sequences known as microsatellites.

More in particular, the invention provides a method for identifying and/or analysing nucleic acid sequences based upon/for polymorphisms associated with such microsatellites.

Further aspects and embodiments will become clear from the further description hereinbelow.

Various methods for analyzing nucleic acid sequences are known in the art. Often, such techniques involve amplification - such as by PCR - of one or more parts of said nucleic acid(s) of a mixture of restriction fragments generated from said nucleic acid(s). The amplified mixture thus obtained is then analysed, e.g. by detection of one or more of the amplified products/fragments. For example, the amplified products/fragments may be separated on the basis of differences in length or molecular weight, such as by gelelectrophoresis, after which the amplified fragments are visualised, e.g. by autoradiography of the labeled amplified fragments or blotting followed by hybridization. The resulting pattern of bands is referred to as a "(DNA-)fingerprint".

Usually in DNA fingerprinting, fingerprints of closely related species, subspecies, varieties, cultivars, races or individuals are compared. Such related fingerprints can be identical or very similar, i.e. contain a large number of corresponding - and therefore less informative - bands.

Differences between two related DNA-fingerprints are referred to as "DNA-polymorphisms". These are amplified fragments - i.e. bands - which are unique in or for a fingerprint and/or for a subset of fingerprints. The presence or absence of such polymorphic bands in a fingerprint - or the pattern thereof - can be used as a genetic marker, for instance to identify a specific species, subspecies, variety, cultivar, race or individual; to establish the presence or absence of a specific inheritable trait and/or of a specific gene; and/or to determine the state of a disease.

For a further discussion of DNA-fingerprinting, DNA-polymorphisms, genotyping, PCR and similar amplification techniques, as well as the techniques and materials used therein, reference is *inter alia* made to the prior art mentioned hereinbelow, as well as to the standard handbooks.

One DNA-fingerprinting technique - which is advantageous in that it requires no prior knowledge of the sequence to be analysed - is selective restriction fragment amplification or AFLP®. In general, AFLP® comprises the steps of:

(a) digesting a nucleic acid, in particular a DNA, with one or more specific restriction endonucleases, to fragment said DNA into a corresponding series of restriction fragments;
(b) ligating the restriction fragments thus obtained with at least one double-stranded synthetic oligonucleotide adapter, one end of which is compatible with one or both of the ends of the restriction fragments, to thereby produce tagged restriction fragments of the starting DNA;
(c) contacting said tagged restriction fragments under hybridizing conditions with at least one oligonucleotide primer;
(d) amplifying said tagged restriction fragment hybridized with said primers by PCR or a similar technique so as to cause further elongation of the hybridized primers along the restriction fragments of the starting DNA to which said primers hybridized; and
(e) detecting, identifying or recovering the amplified or elongated DNA fragment thus obtained.

The AFLP-fingerprint thus obtained provides information on sequence variation in (subsets of) the restriction enzyme sites used for preparation of the AFLP template and the nucleotide(s) immediately adjacent to these restriction enzyme sites in the starting DNA. By comparing AFLP-fingerprints from related individuals, again polymorphic bands (also referred to as "AFLP-markers") can be detected/identified, e.g. for the purposes mentioned hereinabove.

For a further description of AFLP, its advantages, its embodiments, as well as the techniques, enzymes, adapters, primers and further compounds and tools used therein, reference is made to EP-A-0 534 858 and co-pending European applications 98.202.5496 and 98.202.4515, all by applicant.

However, although AFLP® is a very efficient technique for identifying and/or analysing polymorphisms in random subsets of nucleic acid sequences, it cannot be used to analyse nucleic acid sequences for polymorphisms/markers associated with particular sequences such as highly polymorphic microsatellites.

It is known that the genome of many - if not all - eukaryotic organisms contains a large number of repeating nucleotide sequences, which are variously referred to as "simple sequence repeats" or "SSRs"; "simple sequence length polymorphisms" or "SSLPs"; "dinucleotide, trinucleotide, tetranucleotide or pentanucleotide repeats";"(short) tandem repeats" or "STRs"; and/or "*microsatellites*" (the term mainly used in the present description).

It is also known that such microsatellites may provide codominant genetic markers with a high degree of allelic polymorphism. Consequently, microsatellites generally have a higher Polymorphism Information Content (PIC) than bi-allelic markers, such as most AFLP markers. Accordingly, various techniques have been developed in the art to analyse nucleic acid sequences for the presence or absence of such microsatellite-associated polymorphisms/markers.

Usually, these known techniques involve the amplification of the nucleic acid - and/or of a mixture of restriction fragments generated from said nucleic acid - with a combination of (at least) two primers, which either both flank the repeat motif of a microsatellite present in the starting nucleic acid, or of which at least one is (intended to be) complementary to (the sequence of) a microsatellite present in the starting nucleic acid (also referred to as the *"microsatellite-directed primer").*

The amplified mixture thus obtained is then analysed, for instance based upon the differences in size/length of the amplified fragments obtained. Usually, this is carried out using conventional gelelectrophoresis/ autoradiography to provide a fingerprint, which can then be analysed for the presence or absence of the specific polymorphic bands.

Generally, these known techniques differ in the (combination of) primers used to amplify the nucleic acid/ restriction fragments, as will be further discussed hereinbelow.

For a description of some of these known techniques, reference is made to WO 96/22388 by applicant; EP 0 804 618; Wu et al., *Nucl. Acids Research,* 1994, Vol.22., No.15, 3257-3258; Matsumoto et al, *Mammalian Genome 9,* 531-535 (1998); as well as to some of the further prior art mentioned therein. Also, for the sake of convenience, in the description hereinbelow and unless indicated otherwise, essentially the same terms will he used as in the abovementioned art. For instance, unless indicated otherwise, the following terms will have their usual meaning from the references indicated above:

- *"simple sequence repeats"* or "*SSRs*"; "*simple sequence length polymorphisms*"; "*dinucleotide, trinucleotide, tetranucleotide or pentanucleotide repeats*"; "*simple tandem repeats"* and/or "*microsatellites*" (which terms should be considered equivalent and interchangeable);
- *"tandem repeat";*
- *simple SSR*"; "*compound SSR*", *"perfect SSR*", *"imperfect SSR*", "*perfect compound SSR"* (vide for instance page 8 of EP 0 804 618);
- "*non-degenerate*"; "*(fully or partially) degenerate*"; *"arbitrary"* (vide in particular EP 9 804 618, page 10);
- "*Random amplified microsatellite polymorphisms*" or *"RAMP";* and "*RAMP-primer*" (vide for instance Wu et al);
- *"anchor"* or "*anchor region"* (vide for instance page 8 of EP 0 804 618);
- "*AFLP*", "*AFLP-primer*", "*adapter*", *"selective bases/nucleotides*", "*frequent cutter",* "*rare cutter"* (vide for instance EP 0,834,858)
- "*RAPD primer"* (vide for instance page 3 of EP 0 804 618).

It is a general object of the invention to provide a method for the identification and/or analysis of nucleic acid sequences.

In particular, it is an object of the invention to provide a method that can be used to analyse a nucleic acid sequence for one or more microsatellite-associated polymorphisms/markers.

Generally, the above objects are achieved by amplifying said nucleic acid - and in particular one or more adapter-ligated restriction fragments generated from said nucleic acid-with a combination of a RAMP-primer and an AFLP-primer; and then analysing the amplified mixture thus obtained.

Thus, in its broadest scope, the invention comprises the use of at least one RAMP-primer in combination with at least one AFLP-primer in analysing nucleic acid sequences.

In particular, the invention comprises the use of at least one RAMP-primer in combination with at least one AFLP-primer in analysing a nucleic acid sequence for (the presence or absence of) microsatellite-associated polymorphisms/markers.

By "*microsatellite-associated polymorphism/marker*" is generally meant any polymorphism or marker that is caused by, and/or that is related to, the presence and/or absence of a microsatellite in said nucleic acid, e.g. at one or more specific sites in said nucleic acid.

Usually, in the invention, such a presence or absence, respectively, of a microsatellite at such site(s) in the nucleic acid to be analysed (or for instance the presence of a different microsatellite at such site(s) will lead to the generation of different polymorphic bands, for instance bands that correspond to amplified fragments of different size and/or length (so-called "fragment length polymorphisms").

The method of the invention is schematically illustrated in the non-limiting Figure 1, in which the RAMP-primer is indicated as (1), the nucleic acid to be amplified - also referred to hereinbelow as the "*target DNA*" - is indicated as (2), and (the sequence of) a microsatellite present in/on said target DNA is indicated as (3).

As schematically shown in Figure 1, the RAMP-primer (1) is (intended to be) complementary to that part of the sequence of the target DNA (2) that at least comprises the 5' part of the microsatellite repeat sequence (3), so as to allow - e.g. during amplification-the extension of the RAMP-primer (1) in the 3'-direction along the target DNA (2), which serves as a template for said extension of the RAMP-primer (1).

As also schematically shown in Figure 1, the RAMP-primer (1) may be considered to comprise essentially two parts, i.e. a 3'-part and a 5'-part, indicated in Figure 1 as (4) and (5), respectively.

The 3'-part (4) of the RAMP-primer (1) is (intended to be) essentially complementary to (the sequence of) the microsatellite (3). The 5'-part (5) of the RAMP-primer (1) is (intended to be) complementary to the (same number of) bases/nucleotides that, in the target DNA (2), are directly adjacent to the 3'-end of the microsatellite (3). The nucleotides of the 5'-part (5) of the RAMP-primer - which constitute the so-called "*anchor (region)"* of the RAMP-primer (1) and thus will also be referred to hereinbelow as the "*anchor nucleotides"* -are indicated as (6) in Figure 1.

The AFLP-primer used in the invention, indicated as (7) in Figure 1, is essentially the same as a conventional AFLP-primer, in that it is (at least) complementary to (the sequence of) an adapter, indicated as (8) in Figure 1, that has been linked to the target DNA (2), so as to allow - e.g. during amplification - the extension of the AFLP-primer (7) in the 3'-direction along the target DNA (3), which serves as a template for said extension of the AFLP-primer (7). Most preferably, as in AFLP®, said primer contains, at its 3'-end, a number of so-called "selective bases/nucleotides" - indicated as (9) in Figure 1 - that are (intended to be) complementary to (same number of) bases/nucleotides that, in the target DNA (2), are directly adjacent to the 3' end of the adapter (8).

Using the RAMP-primer (1) and the AFLP-primer (7), the target nucleic acid (2) is amplified, e.g. as indicated by the arrows in Figure 1. In particular, during said amplification, the RAMP-primer (1) will be extended along one strand of the (double stranded) target DNA (2) and the AFLP-primer will be extended along the other strand of the (double stranded) target DNA (2), e.g. so as to allow for efficient/exponential amplification. The mixture of amplified products/fragments thus obtained may then be analysed, e.g. by detecting/ visualizing at least one, and up to essentially all, of the amplified products/fragments, e.g. as further described hereinbelow.

Accordingly, if- for instance - there is a CT/GA type repeat in an AFLP fragment generated with for example *EcoRI* and *TaqI* as restriction enzyme combination, according to the invention said fragment may be amplified exponentially and then visualized in either of two ways, i.e. 1) using a RAMP primer directed against the CT repeats in combination with a TaqI (of EcoRI) AFLP primer; or 2) using a RAMP primer directed against the GA repeats in combination with an EcoRI (of TaqI) AFLP primer. The length(s) of the amplified products obtained in 1) and 2) respectively will depend upon the "distance" of the repeat to the respective restriction site(s).

In the state of the art, a method for analysing nucleic acid sequences for microsatellite associated polymorphisms/markers involving the use of both a RAMP-primer and an AFLP-primer has not yet been described or suggested.

For instance, Wu et al. describe the use of a RAMP-primer in combination with a RAPD-primer. Such an RAPD primer differs from an AFLP-primer in that it is not (intended to be) complementary to an adapter. Thus, in the method of Wu et al, the target DNA will usually also not contain an adapter.

EP 0 804 618 describes a technique referred to as "SAMPL". This technique involves the use of two primers, one of which may *inter alia* be an AFLP-primer, and the other of which - the microsatellite-directed primer also referred to as the "SAMPL"-primer- is (intended to be) complementary to a so-called "*compound repeat SSR",* which is an SSR comprising at least two different parts with each part comprising a different repeated sequence (e.g. 5'-CTCTCTCTGAGAGAGA-3').

However, even though the latter primer is intended to be complementary to a microsatellite (albeit a specific type of microsatellite) and even though said primer may also be considered to comprise a 3'-part and a 5'-part, the microsatellite-directed primer used in EP 0 804 618 differs from the RAMP-primer used in the invention.

In particular, in the microsatellite-directed primer of EP 0 804 618, both the 3'-part of the primer as well as the 5'-part of the primer is (intended to be) complementary to the microsatellite, i.e. the sequence of the compound repeat SSR. In particular, the 3'-part is (intended to be) complementary to a first repeat/part of the compound repeat SSR, whereas the 5'-part is (intended to be) complementary to a second repeat/part of the compound repeat SSR.

Thus, the microsatellite-directed primers used in EP 0 804 618 will themselves essentially consist only of repeats of dinucleotides, trinucleotides, etc.. Accordingly, these primers will essentially not contain bases/nucleotides that are (intended to he) complementary to bases/nucleotides that lie immediately adjacent to the 5' boundery of (the sequence of) the microsatellite as present in/on the target DNA; or at least the presence of such nucleotides is not required.

In this respect, EP 0 804 618 *inter alia* states (page 11, lines 15-18): "*Additionally, PCR primers representing these compound SSR sequences are self-anchoring, such that the 5'-most repeat serves as the anchor for primer extension by the 3'-most of the two repeats, thus obviating the need to incorporate into these primers any additional degenerate or fixed sequences as 5' or 3' flanking regions".*

As an alternative for the use of primers that are complementary to compound repeat SSRs, EP 0 804 618 on page 14, lines 49-56 also describes the use of primers of which the 3'-part corresponds to a simple SSR or microsatellite, whereas the 5'-part of such a primer "*contains 3 to 5 fully or partially degenerate nucleotides, which serve to anchor the primer adjacent to a microsatellite in the targeted genome".*

In this respect, as indicated on page 10 of EP 0 804 618: " "*Fully degenerate*" *indicates the presence of an equal mixture of the four possible nucleotide bases (A, G, C or T) at a particular nucleotide position, partially degenerate*

*indicates the presence of only two or three of the four possible bases at a particular base position."*

In contrast, the 5'-part of the primer(s) used in the invention contain(s) - in the terms used in EP 0 804 618 - only "non-degenerate" bases/nucleotides, which refers to *"the occurrence of a single, specified nucleotide type at a particular position or at multiple specified positions in the linear ordering of nucleotides*". A particular advantage of the use of such defined (i.e. completely non-degenerate) anchor sequences is that RAMP primers directed at the same repeat motif (identical 3' sequence of the primer) but with different anchor sequences (5' prime sequence of the primer) can be used to target completely distinct subsets or partially overlapping subsets of microsatellites. This characteristic of the invention increases dramatically the number of different microsatellite repeats that can be identified compared to the SAMPL technique described in EP 0 804 618. Thus the resolving power of the method of the invention to visualize microsatellites compares favorably to that of the art described in EP 0 804 618.

Also, according to the definitions given in EP 0 804 618 "*Any nondegenerate nucleotide position can carry an intended base (either A, G, C or T) that is known for example to correspond to a given template site, or it can carry an arbitrarily chosen base, which will correspond to a target site that is not known a priori".* Both these possibilities are also incorporated within the scope of the present invention, with the use of arbitrarily chosen bases/nucleotides in the RAMP-primers of the invention usually being preferred.

However, according to one particular embodiment, if the "anchor" sequences in the RAMP-primer are non-arbitrary, said anchor sequences do not form or correspond to a repeat motif ( or in other words, the RAMP-primer used does not correspond to a "SAMPL"-primer. i.e. as used in EP 0 804 618)

Thus, in a first aspect, the invention relates to the use of (the combination of) at least one RAMP-primer and at least one AFLP-primer in amplifying a nucleic acid sequence (herein also referred to as the "*target nucleic acid*").

In this aspect of the invention, and with reference to Figure 1, the target nucleic acid usually will comprise at least one adapter (8) and at least one further nucleic acid sequence, indicated as (11) in Figure 1, to which said adapter has been ligated.

In particular, in this aspect of the invention, the further nucleic acid sequence (11) present in the target nucleic acid (2) will be a restriction fragment. For instance, said further nucleic acid (11) can be a restriction fragment derived from a starting DNA - including but not limited to genomic DNA, cDNA or recombinant DNA such BAC DNA, cosmid DNA or plasmid DNA - by restriction with at least one restriction endonuclease (as further described hereinbelow), although the invention in its broadest sense is not limited thereto.

Also, the target nucleic acid (2) will usually be a DNA sequence, and in particular a double stranded DNA sequence, although the invention in its broadest sense is again not limited thereto.

The target nucleic acid (2) may comprise a single adapter (8) but usually comprises two adapters (8), e.g. each ligated to one end of the restriction fragment (11) present in the target nucleic acid. Also, when two adapters (8) are present, they may be the same or different.

Also, the target nucleic acid (2) may be part of a mixture of such target nucleic acids. For instance, when the target nucleic acid comprises a restriction fragment ligated to at least one adapter, it may be part of a mixture of such adapter-ligated restriction fragments. Such a mixture may for instance be obtained by ligating at least one adapter to a mixture of restriction fragments, which may be carried out in a manner known per se, for instance as described in the above prior art, including but not limited to EP 0 834 858.

Optionally, such a mixture of target nucleic acids may (already) have been subjected to a (pre)amplification step, i.e. prior to the amplification with the RAMP-primer and the AFLP-primer. This may also be/have been a "selective" preamplification for reducing the complexity of the mixture. For instance, when the target nucleic acid(s) (2) contain two adapters (8), such a selective preamplification may be carried out as a conventional AFLP-preamplification, for which reference is made to EP 0 834 858.

Besides the adapter, the target nucleic acid (2) preferably also contains (or is at least suspected to contain) a microsatellite, or otherwise the target nucleic is at least part of a mixture of such target nucleic acids of which at least one target nucleic acid contains (or is suspected to contain) a microsatellite; and in particular a microsatellite to which the RAMP-primer (1) can and/or is intended to hybridize.

The microsatellite (3) can be any microsatellite/SSR or sequence corresponding thereto, and in particular can be any microsatellite/SSR that may occur naturally in the genome of an eukaryote. Some non-limiting examples of (nucleotide sequences of) such microsatellites are described in the abovementioned art.

As such, the microsatellite/SSR may for instance be a simple SSR or a compound SSR; and/or may be a perfect SSR and/or an imperfect SSR; or any combination thereof. Preferably, the microsatellite is a multi-allelic SSR, although the invention in its broadest sense is not limited thereto.

The at least one RAMP-primer (1) will be at least such that, when (the sequence of) a microsatellite (3) to which said RAMP-primer (1) is complementary is present in the target nucleic acid, it is capable of hybridizing with said microsatellite (3) so as to allow extension of the RAMP-primer (1) along the target nucleic acid (2)

Usually, a RAMP-primer (1) used in the invention will contain a total of between 8 and 20 nucleotides, and in particular between 12 and 16 nucleotides.

Of these, between 4 and 10 nucleotides, and in particular between 6 and 8 nucleotides will form part of the 3'- part (4) of the RAMP-primer (1) - i.e., the part that is complementary to (the repeat motif of) the microsatellite - and between 4 and 10 nucleotides, and in particular between 6 and 8 nucleotides will form part of the 5'- part (5) of the RAMP-primer (1), i.e. the anchor region.

Thus, with reference to the symbols used on pages 9 and 10 of WO 96/22388, a RAMP-primer suitable for use in the invention may for example be schematically represented by the formula

$$H_m\text{-}[X'Y']_n$$

in which H represents an anchor nucleotide, arbitrarily selected from the four bases A,T, C or G; "m" is a whole number between 1 and 10, preferably between 3 and 8; X'Y' represents a simple sequence repeat unit of 1, 2, 3 or 4 nucleotides intended to be complementary to a simple sequence repeat XY unit present in a microsatellite in the target nucleic acid; $[X'Y']_n$ represents a simple sequence repeat of n repeat units; and "n" is a whole number between 2 and 10 , preferably between 3 and 6. Thus, in the above formula, and with reference to Figure 1 and the description hereinabove, $H_m$ represents 5'-part (5) of the RAMP-primer, whereas $[X'Y']_n$ represents the 3'-part (4).

Thus, usually, in the invention, (the 3'-part of) the RAMP primer will represent a simple sequence repeat $[X'Y']_n$.

Also, with reference to the terms used in EP 0 804 618, the anchor nucleotides that form the 5'-part (5) of the RAMP-primer will be *"non-degenerate"* nucleotides, and preferably are "*arbitrary*" non-degenerate nucleotides. As mentioned above, (the use of) such microsatellite-directed primers is not disclosed by EP 0 804 618.

The at least one AFLP-primer (7) will be essentially the same as a conventional AFLP-primer, e.g. as described in EP 0 834 858, and will generally contain a constant region - indicated as (10) in Figure 1 - and one or more selective nucleotides at the 3'-end thereof.

Also, the at least one AFLP-primer (7) is most preferably essentially complementary to at least one of the adapters (8) used, e.g. so as to allow extension of the AFLP-primer (7) along the target nucleic acid (2).

Preferably, the AFLP-primer (7) will contain a total of between 15 and 50 nucleotides, and in particular between 18 and 30 nucleotides. Also, preferably, the AFLP-primer (7) will contain between 1 and 6, preferably between 1 and 3 selective nucleotides.

The amplification of the target nucleic acid (2) with the at least one RAMP-primer (1) and the at least one AFLP-primer (7) may be carried out under conditions known per se, including but not limited to conditions known per se for amplification using RAMP-primers and/or conditions known per se for amplification using AFLP-primers. Such conditions are for instance described in the abovementioned prior art (e.g. Wu et al. for RAMP-primers and EP 0 834 858 for AFLP-primers) and some non-limiting examples of suitable conditions are also given in the Experimental Part hereinbelow. It is envisaged that based upon these disclosures, the skilled person will be able to select (a range of) optimal conditions for the amplification of a given (mixture of) target nucleic acid(s) with a given combination of RAMP-primer and AFLP-primer.

Usually, the amplification is carried out using only one RAMP-primer as described above and only one AFLP-primer as described above, although the invention in its broadest sense is not limited thereto.

Also, the at least one RAMP-primer and the at least one AFLP-primer are preferably such that they allow for efficient/exponential amplification. In this respect, it should be noted that, when the target nucleic acid is part of a mixture of such target nucleic acids, in the amplification step usually more than one of the target nucleic acids that are present is said mixture will be amplified, i.e. to provide a mixture of amplified fragments.

After the amplification with the RAMP-primer and the AFLP-primer, the at least one amplified nucleic acid thus generated is detected. For instance, when the amplification step has provided a mixture of amplified fragments as described hereinabove, one or more - and up to essentially all - amplified fragments present in said mixture may be detected.

Said detection may be carried out using any technique known per se for the detection of an amplified nucleic acid/fragment and/or for analysing a mixture of amplified nucleic acids/fragments. Suitable techniques are described in the abovementioned art and for instance include techniques in which the amplified fragments are separated and visualized (e.g. gelchromatography and autoradiography to provide a fingerprint); (other) detection techniques based upon the mass and/or the size of the amplified fragments; and techniques involving the hydridization of one or more of the amplified fragments to a complementary nucleotide sequence (in which said complementary nucleotide sequence may for instance be immobilized on a suitable carrier, e.g. as part of an array of such nucleotide sequences) followed by detection of such hybridization events. Generally, these detection techniques will be such that they allow for the detection of polymorphisms, as further described below.

In another aspect, the invention relates to a method for analysing a nucleic acid sequence, said method at least comprising the steps of:

(a) amplifying at least one restriction fragment generated from the nucleic acid to be analysed, in which said restriction fragment has been ligated to at least one adapter, with at least one RAMP-primer and at least one AFLP-primer to provide at least one amplified nucleic acid sequence;
and optionally comprising the further step of:
(b) detecting at least one of the amplified nucleic acid sequences thus obtained.

More specifically, this aspect of the invention relates to a method for analysing a nucleic acid sequence, said method comprising the steps of:

(a) restricting the starting nucleic acid with at least one restriction endonuclease to provide a mixture of restriction fragments;
(b) ligating the restriction fragments thus obtained to at least one adapter;
(c) amplifying the mixture of adapter-ligated restriction fragments thus obtained with at least one RAMP-primer and at least one AFLP-primer to provide a mixture of amplified restriction fragments;
and optionally comprising the further step of:
(d) detecting at least one of the amplified restriction fragments thus obtained.

The invention also relates to the use In the above aspects of the invention, the (starting) nucleic acid is preferably a DNA sequence, more preferably a double stranded DNA sequence.
In particular, the starting nucleic acid can be a nucleic acid that contains (or is at least suspected to contain) a microsatellite to which the RAMP-primer used can and/or is intended to hybridize. For instance, the starting nucleic acid sequence can be genomic DNA, cDNA; and in particular eukaryotic genomic DNA, cDNA or (a mixture or a library of) recombinant DNA clones, e.g. derived from a plant, animal or a human.
For instance, the starting nucleic acid can be derived from agronomically important crops such as wheat, barley, maize, tomato, pepper, lettuce, rice, soybean etc. ; from animals such as such as mouse, rat, pig, chicken, fish, etc.; and/or from humans.
In the restriction step a), the starting nucleic acid is restricted with at least one restriction endonuclease, which may be any suitable restriction endonuclease, including but not limited to those mentioned below.
In particular, the starting nucleic acid may be restricted with two different restriction endonucleases. For instance, the starting nucleic acid may be restricted with one "frequent cutter" restriction endonuclease, which serves the purpose of reducing the size of the restriction fragments to a range of sizes which are amplified efficiently; and one "rare cutter" restriction endonucleas, which serves the purpose of targeting rare sequences. For both, reference is made to for instance EP-A-0 534 858 and EP-A-0 721 987 by applicant, incorporated herein by reference.
Some non-limiting examples of suitable frequent cutter enzymes are *Mse*I and *Taq*I. Some non-limiting examples of commercially available rare cutters are *Pst*I, *Hpa*II, *Msp*I, *Cla*I, *Hha*I, *EcoR*II, *Bst*BI, *Hin*P1, *Mae*II, *Bbv*I, *Pvu*II, *Xma*I, *Sma*I, *Nci*I, *Ava*I, *Hae*II, *Sal*I, *Xho*I and *Pvu*II, of which *Pst*I, *Hpa*II, *Msp*I, *Cla*I, *EcoR*II, *Bst*BI, *Hin*P1 and *Mae*II are preferred.
After the restriction step a), the restricted fragments thus obtained are ligated to at least one adapter. This adapter will be essentially the same as the adapter(s) used in conventional AFLP, for which reference is again made to the prior art relating to AFLP mentioned above.
As in conventional AFLP, the at least one adapter used is most preferably such that it is suitable for use with at least one of the restriction enzymes used in the restriction step a). For instance, when the starting DNA is restricted with two restriction endonucleases ( e.g. a frequent cutter and a rare cutter) preferably also two adapters are used, each suitable for use with one of the restriction endonucleases.
After the at least one adapter has been ligated with to the restriction fragments, the mixture of adapter-ligated restriction fragments thus obtained may then (directly) be amplified in step c) with the RAMP-primer and the AFLP-primer. However, as already indicated above, prior to said amplification step c), the (adapter-ligated) restriction fragments may first be subjected to a pre-amplification, and in particular a selective pre-amplification for reducing the complexity of the fragment mixture. For instance, such a selective preamplification may be carried out analogous to a selective preamplification known per se from AFLP, for which reference is again made to the prior art related to AFLP mentioned above.
More generally, the restriction step a), the ligation step b) and any preamplification step described above may be carried out in essentially the same manner as the restriction, ligation and amplification steps of conventional AFLP methodology, e.g. according to known AFLP protocols.
The subsequent amplification step c) of the adapter-ligated restriction fragments with the RAMP-primer and the AFLP-primer may be carried out as described hereinabove and as illustrated in Figure 1, in which the adapter-ligated restriction fragments serve as the "target nucleic acid" (2).
Thereafter, the amplified mixture thus obtained is analysed, which is also carried out as described hereinabove.

Generally, these detection techniques will be such that they allow for the detection of polymorphisms, e.g. detectable signals that are unique for the starting nucleic acid. For instance, such a unique detectable signal may be a unique band in a fingerprint or a unique hybridisation event/signal on an array; or the lack of such a band or hybridisation signal.

For this purpose, the detectable signal(s) generated for a specific starting nucleic acid will usually be compared to the detectable signal(s) obtained for one or more related starting nucleic acid(s) under essentially the same conditions (e.g. the use of the same restriction enzymes, adapters, preamplification (if any), RAMP-primer, AFLP-primer and detection technique), for instance by comparing fingerprints and/or hybridization signals/patterns on a given array.

Such related starting nucleic acids may for instance have derived from the same individuals and/or from one or more closely related individuals (e.g. from the same family, genus, species or even variety). For instance, one or more such related starting nucleic acids may be used/incorporated as reference sample(s) in the method of the invention, in which case the results for the starting nucleic acid sequence and the reference sample(s) may be directly compared. Alternatively, the results obtained for a given starting nucleic acid may be compared to results generated earlier for one or more related nucleic acid sequences, which may for instance be part of a database.

Again, such detection techniques and techniques for analysing/comparing the results obtained will be essentially analogous to the techniques used to analyse the results obtained using AFLP, for which again reference is made to the prior art related to AFLP mentioned above.

Thus, from the above, it will be clear that the method of the invention may conveniently be carried out analogous to a conventional AFLP-amplification, in which for the "main" amplification a combination of one AFLP-primer and one RAMP-primer is used, instead of two AFLP-primers.

Also, as in conventional AFLP, the selective nucleotides (9) of the AFLP-primer (7) may be selected arbitrarily. Also, in the RAMP-primer (1), both the anchor nucleotides (6) that form the 5'-part (5) of the RAMP-primer, as well as the specific repeat sequence that forms the 3'-part (4) of the RAMP-primer may be arbitrarily selected, in which said repeat sequence may be chosen arbitrarily from any and all nucleotide sequences that are complementary to the sequence of a naturally occuring microsatellite.

In particular, the repeat sequence that forms the 3'-part (4) of the RAMP-primer (1) may be arbitrarily selected from any and all nucleotide sequences that are complementary to the sequence of a microsatellite that may be present in the starting DNA. In this respect, it should be noted that, generally, any given starting DNA will contain a large number of different microsatellites, e.g. up to thousands or more.

Thus, as with conventional AFLP, the method of the invention does not require any prior knowledge of the sequence to be analysed, nor the use of any specifically designed primers.

Also, the invention may allow the detection of microsatellite-associated polymorphisms/markers in conjunction with AFLP-markers, and thus provide a very powerful (combined) technique for the analysis of a starting nucleic acid for both these typese of highly informative genetic markers.

However, as with conventional AFLP, it may be that some (combinations of) specific AFLP-primers and RAMP-primers may provide, for a specific starting DNA, more informative results (e.g. more polymorphic bands) than other combinations, and that some combinations may even provide no informative results at all. Nevertheless, based upon the disclosure herein, the skilled person will be able to provide one or more suitable combinations of a RAMP-primer and an AFLP-primer for analysing a specific starting DNA according to the method of the invention, optionally after some preliminary experiments and/or a limited degree of trial and error.

In principle, the method of the invention can be used for any application for which a microsatellite-associated polymorphic marker can be developed or used. Such applications include, but are not limited to, all uses described in the abovementioned art, e.g. in WO 96/22388 by applicant and/or in EP 0 804 618, such as in genotyping, genetic mapping, genetic profiling and DNA-identification techniques, e.g. to identify a specific species, subspecies, variety, cultivar, race or individual, to establish the presence or absence of a specific inheritable trait and/or of a gene; or to determine the state of a disease.

Generally, and in particular for the above applications, the method of the invention may provide the following advantages (e.g. compared to the prior art methods for detecting microsatellite-associated polymorphisms mentioned hereinabove:

- efficient targeting of a large proportion of microsatellites present in the genome, particularly those with simple repeat motifs.
- highly reproducible fingerprint patterns due to excellent reproducibility of the AFLP technique compared to other techniques

A particularly envisaged application comprises for instance amplification of several highly informative microsatellites in combination with AFLP markers in multiplex form in a single amplification reaction

It is also envisaged that one or more of the microsatellite-associatcd markers identified using the method of the invention may be (further) developed into "classical" PCR-test. This may for instance be carried out by a method as schematically illustrated in the non-limiting Figure 2.

Generally, this method involves the identification a microsatellite-associated polymorphic band or fragment, e.g. as described hereinabove. This polymorphic band 11 (e.g. a band amplified using the combination of a RAMP-primer and an AFLP-primer for the first restriction enzyme used for AFLP template preparation) and optionally one or more alleles thereof) is then isolated (e.g. cut out of the gel obtained after gelelectrophoresis) and sequenced (step 1 in Figure 2). Based upon the sequence of the polymorphic band(s) thus obtained, a suitable PCR-primer may be selected/designed from the sequence flanking the microsatellite repeat sequence at the 3' end. Next, this PCR primer, in combination with an AFLP primer corresponding to the second enzyme used for AFLP template preparation is used to amplify a fragment that contains the microsatellite and the 5' flanking sequence, which is not included in the polymorphic band initially chosen for sequencing (step 2). From this 5' flanking sequence a suitable second PCR primer may be selected/designed (step 3), which together with the first PCR primer matching the 3' flanking sequence can then be used in a conventional PCR-detection, e.g. on the starting DNA (step 4).

Furthermore, it is also envisaged that one or more of the microsatellite-associated polymorphic bands or fragments identified by the method of the invention may be isolated and optionally sequenced, and may then be used to generate a nucleotide sequence representative for said microsatellite-associated marker for use in - for instance - an array for the analysis of nucleic acid sequences. Such arrays and their preparation are known per se in the art, for instance from applicants pending application PCT/NL99/00743 entitled "array and method for analysing nucleic acid sequences", filed 3-12-1999.

In yet another aspect, the invention relates to the use of a RAMP-primer in the method described hereinabove. The invention also relates to the use of an AFLP-primer in the method described hereinabove.

In another aspect, the invention relates to the use of (the combination of) at least one RAMP-primer and at least one AFLP-primer in analysing a nucleic acid sequence.

In particular, this aspect of the invention relates to the use of (the combination of) at least one RAMP-primer and at least one AFLP-primer in analysing a nucleic acid sequence for (the presence or absence of) polymorphisms/markers associated with microsatellites.

Yet another aspect comprises any data generated by the method of the invention, optionally on a suitable data carrier, such as paper or a computer disk. Such data may for instance include the generated DNA-fingerprints (e.g. in the form of a gel) and/or autoradiographs/photographs or other reproductions thereof, as well as (stored) analog or digital data thereon, e.g. in the form of a database.

The invention also comprises kits for use in the invention, said kits at least comprising a RAMP-primer and an AFLP-primer; and usually also comprising an adapter complementary to said AFLP-primer. These kits can further contain any known component for such kits, including but not limited to components known per se for AFLP kits, such as restriction enzymes (in which case the adapters are preferably suited to be ligated to the restrictes sites generated with said enzyme); a polymerase for amplification, such as *Taq*-polymerase; nucleotides for use in primer extension; as well as buffers and other solutions and reagentia; manuals, etc.. Further reference is made to the European patent application 0 534 858, incorporated herein by reference.

The invention will now be illustrated by means of the following non-limiting Experimental Part and the Figures, in which:

- Figures 1 and 2 are schematic representations of the method of the invention;
- Figure 3 shows a section in the 200-280 basepairs fragment mobility size range of the fingerprint patterns obtained from lettuce lines Rachel (lane 1), Vicky (lane 2), Lianne (lane 3), Quincy (lane 4), Thirza (lane 5), Veraly (lane 6), Clarion (lane 7) and Ballora (lane 8) using the method of the invention, as further described in Example 1;
- Figure 4 shows a section in the 270-400 basepairs fragment mobility size range of the fingerprint patterns obtained from the parental lines and six F2 progeny of a pickle mapping population using the method of the invention, as further described in Example 2;
- Figure 5 shows a section in the 180-330 basepairs fragment mobility size range of the fingerprint patterns obtained from the parental lines and 32 F2 progeny of maize mapping population using the method of the invention, as further described in Example 3;
- Figure 6 shows a section in the 105-110 basepairs fragment mobility size range of the fingerprint patterns obtained from the parental lines and 26 F2 progeny of pepper mapping population using the method of the invention, as further described in Example 4;
- Figure 7 shows a section in the 250-500 basepairs fragment mobility size range of the fingerprint patterns obtained from the parental lines and 6 F2 progeny of a maize mapping population using the method of the invention, as further described in Example 5;
- Figure 8 shows the lettuce (SEQ ID Nos.11-13), pickle (SEQ ID Nos. 22-23), maize (SEQ ID Nos.30-31) and pepper (SEQ ID Nos. 34-35) used in the Examples below.

Experimental Part:

Example 1: Targetting of microsatellite repeat polymorphisms in lettuce using the combination of an AFLP primer and a RAMP primer.

1. Description of biological materials

The biological materials used are lettuce lines Rachel, Vicky, Lianne, Quincy,Thirza, Veraly, Clarion and Ballora.

2. TaqI/MseI AFLP template preparation, preamplification and amplification using a selective TaqI AFLP primer in combination with a RAMP primer

AFLP templates prepared using the restriction enzymes TaqI and MseI, and preamplification reactions were carried out according to standard procedures described by Vos et al., (Nucleic Acids Research 23: no 21, pp. 4407-4414, 1995; and patent application EP0534858).

Microsatellite-repeat fragment-enriched fingerprints patterns were obtained by carrying out a selective amplification from a 20-fold diluted +0/+0 preamplification mixture using the combination of a TaqI AFLP selective +3 AFLP primer and a RAMP primer. The amplification reactions were carried out according to the amplification conditions described by Vos et al and in EP0534858. For the results shown in Figure 3, RAMP primer 98L76 directed at CT sequences (left hand side of Figure 3) or RAMP primer 99A54 directed at GA sequences (right hand side of the Figure 3) were used in combination with AFLP primer TR48. The RAMP primers 98L76 or 99A54 were end-labeled with $^{33}P$ and the amplification mixtures were resolved on a standard AFLP (sequence) gel. Gels were processed and exposed to a phosphor-imaging screen as described (Vos et al., 1995) to generate the gel-image.

The sequences of the adapters, AFLP preamplification primers and (selective AFLP) amplification primers used are as follows:

MseI adapter:

92A18: 5'-GACGATGAGTCCTGAG-3' (SEQ ID No.1 )

92A19: 3'-TACTCAGGACTCAT-5' (SEQ ID No.2 )

MseI + 0 preamplification AFLP primer:

M00L: 5'-GACGATGAGTCCTGAGTAA-3' (SEQ ID No.3 )

TaqI (rare) adapter:

96A22: 5'CTCGTAGACTGCGTAC-3' (SEQ ID No.4 )

96A23: 3'-TCTGACGCATGGC-5' (SEQ ID No.5 )

TaqI + 0 preamplification AFLP primer:

T00K: 5'-GTAGACTGCGTACCGA-3' (SEQ ID No.6 )

TaqI +3 selective amplification AFLP primer:

TR48: 5'-GTAGACTGCGTACCGACAC-3' (SEQ ID No.7 )

RAMP amplification primers:

98L76: 5'-GATAAGCGCTCTCTCT-3' (SEQ ID No.8 )

99A54: 5'-GACGGCACGAGAGAGA-3' (SEQ ID No.9 )

3. Results

Figure 3 shows a section in the 200-280 basepairs fragment mobility size range of the fingerprint patterns obtained after separating the amplified fragments on a sequence gel. The fingerprints on the left hand side of the Figure 3 were obtained with primer combination 98L76/TR48 from lettuce lines Rachel (lane 1), Vicky (lane 2), Lianne (lane 3), Quincy (lane 4), Thirza (lane 5), Veraly (lane 6), Clarion (lane 7) and Ballora (lane 8), as described above.

The fingerprints on the right hand side of the Figure 3 were obtained with primer combination 99A54/TR48 from the same lettuce lines, loaded on the gel in the same order. The bands indicated by arrows may represent alleles of polymorphic microsatellite repeat loci targetted by the RAMP primers used.

4. Confirmation of targetting a microsatellite repeat by nudeotide sequencing

The three bands at the top of the right hand part of Figure 3, amplified using primer combination 99A54/TR48 and indicated by arrows and a star (*) at the side, may represent three alleles of a multi-allelic microsatellite repeat. In order to demonstrate this, the nucleotide sequences of these fragments were determined after excision from the appropriate positions from the sequence gel and eluting the DNA in TE (10 mM Tris, pH 8.0, 1mM EDTA).

The fragments were re-amplified by PCR from the eluates of Quincy (lane 4), Thirza (lane 5) and Clarion (lane 7), using the primers 99A54 and 99G20 according to standard procedures. The PCR products were purified using Qiagen PCR purification kits (Qiagen). Cycle sequencing was performed using a commercially available dye terminator cycle sequencing kit (ABI), according to instructions provided by the manufacturer

99G20 (SEQ ID No.10):

5'-AGCGGATAACAATTTCACACAGGACACACTGGTATAGACTGCGTACCGA-3'

The resulting three sequences, truncated at the TaqI site, are given in SEQ ID Nos 11-13 and are also shown in Figure 8, i.e.:

SEQ ID No.11: 260 basepairs, corresponding to "top" of three bands (Quincy)
SEQ ID No.12: 256 basepairs, corresponding to the "middle" of three bands (Clarion)
SEQ ID No.13: 254 basepairs, corresponding to the "bottom' of three bands (Thirza)

5. Conclusion

From Figure 3 and the inspection of the sequences of SEQ ID NOs 11-13 and Figure 8 it is apparent that these sequences differ only a the number of CT repeats but are otherwise identical. Furthermore these CT-repeat sequences are flanked by the (inverse complement) sequence of RAMP primer 99A54. These observations prove that a CT microsatellite repeat polymorphism is targetted by using RAMP primer 99A54 in combination with AFLP primer TR48 and that it is very likely that the three bands observed on the fingerprint shown in Figure 3 represent three alleles of the same microsatellite locus.

Example 2: Targetting of potential microsatellite repeat polymorphisms in pickle using the combination of an AFLP primer and a RAMP primer.

1 Description of biological materials

The biological materials used are the parental lines and six F2 progeny of a pickle mapping population. The parental lines are G421 (indicated by P1 in Figure 4) and H19 (indicated by P2 in Figure 4). The F2 progeny used are numbered 001, 002, 003, 004, 005 and 006.

2. EcoRI/MseI template preparation and amplification using a selective EcoRI primers in combination with a RAMP primer.

AFLP templates prepared using the restriction enzymes EcoRI and MseI and preamplification reactions were carried out as described in Example 1 or references incorporated therein. Microsatellite-repeat fragment-enriched fingerprints patterns were obtained by carrying out a selective amplification from 20-fold diluted +0/+0 preamplification mixture using the combination of a EcoRI AFLP selective +1 AFLP primer and a RAMP primer. The amplification reactions were carried out as described in Example 1. The results are shown in Figure 4.

EcoRI+ 1 primers E01K (left hand side of Figure 4) or E02K (right hand side of the Figure 4) were used in combination with RAMP primer 98L33, which is directed at CA-repeat sequences. RAMP primer 98L33 was end-labeled with $^{33}P$. The amplification mixtures were resolved on an AFLP (sequence) gel, which was run, processed and exposed as described in Example 1.

The sequences of the adapters, AFLP preamplification primers and (selective AFLP) amplification primers used are as follows:

EcoRI adapter:

91M35: 5'-CTCGTAGACTGCGTACC-3' (SEQ ID No.14)
91M36: 3'-CTGACGCATGGTTAA-5' (SEQ ID No.15)

EcoRI + 0 preamplification AFLP primer:

E00L: 5'-GTAGACTGCGTACCAATTC-3' (SEQ ID No.16)

EcoRI AFLP primer sequences:

E01K: 5-GACTGCGTACCAATTCA-3' (SEQ ID No.17)
E02K: 5-GACTGCGTACCAATTCC-3' (SEQ ID No.18)

MseI adapter: (see example 1)

MseI+ 0 preamplification AFLP primer:

M00K 5'-GATGAGTCCTGAGTAA-3' (SEQ ID No.19)

RAMP amplification primer:

98L33: 5'-CTCGATTTACACACAC-3' (SEQ ID No.20)

3. Results

Figure 4 shows a section in the 270-400 basepairs fragment mobility size range of the fingerprint patterns obtained after separating the amplified fragments on a sequence gel. The fingerprints on the left-hand side of Figure 4 were obtained with primer combination E01K/98L33 from pickle parental lines G421 (P1), H19 (P2) and six F2 offspring 001, 002, 003, 004, 005 and 006. The six F2s were loaded in lanes 1 through 6, respectively. The fingerprints on the right hand side of Figure 4 were obtained with primer combination E02K/98L33 from the same pickle lines, applied to the gel in the same order. The bands indicated by arrows may represent alleles of polymorphic microsatellite repeat loci, targetted by the RAMP primer used. Figure 4 also demonstrates that different fingerprint patterns are obtained when the same RAMP primer is used in combination with a different selective AFLP primer, as would be expected from selective amplification with (an) AFLP primer(s).

4. Confirmation of targetting a microsatellite repeat by nucleotide sequencing

The two bands at the top right hand part of Figure 4, amplified using primer combination E02K/98L33 and indicated by arrows and a star (*) at the side, may represent two alleles of a microsatellite repeat locus. In order to demonstrate this, the nucleotide sequences of these fragments were determined after excision of the bands in G421 (P1) and H19 (P2) and re-amplification with the primers 98A28 and 98L33 according to the procedures described in Example 1.

98A28 (SEQ ID No.21 ):

5'-AGCGGATAACAATTTCACACAGGATAGACTGCGTACCAAT-3'

The two resulting sequences, truncated at the EcoRI site, are given in SEQ ID NOs. 22-23 and in Figure 8, i.e:

SEQ ID No.22: 373 basepairs: "upper" band (G421)
SEQ ID No.23: 357 basepairs: "lower" band (H19)

5. Conclusion

From Figure 4 and inspection of the sequences of SEQ ID Nos 22 and 23 and Figure 8 it is apparent that the length difference between these fragments results from a 10 basepairs insertion/deletion polymorphism located in the internal sequence of the fragment and a 6 basepair insertion/deletion polymorphism at the boundary of a GT microsatellite-repeat. This microsatellite-repeat is targetted at its opposite boundary by RAMP primer 98L33. Thus, it is very likely that these two sequences are allelic sequences derived from the same microsatellite-repeat locus.

Example 3: Targetting of microsatellite repeat polymorphisms in maize using the combination of an AFLP primer and a RAMP primer.

1. Description of biological materials

The biological materials used are the parental lines and 32 F2 progeny of maize mapping population. The parental lines are A7 (indicated by P1 in Figure 5) and B73 (indicated by P2 in Figure 5). The F2 progeny used are numbered 001-032.

2. PstI/MseI AFLP template preparation, preamplification and amplification using a selective PstI primer in combination with a RAMP primer.

AFLP templates prepared using the restriction enzymes PstI and MseI and preamplification reactions were carried out as described in Example 1 or references incorporated therein. Microsatellite-repeat fragment-enriched fingerprints patterns were obtained by carrying out a selective amplification from 20-fold diluted +0/+0 preamplification mixture using the combination of a PstI AFLP selective +2 AFLP primer and a RAMP primer. The amplification reactions were carried out as described in Example 1.

For the results shown in Figure 5, PstI + 2 primer P16 was used in combination with RAMP primer 98L39, which is directed at GA-repeat sequences. RAMP primer 98L39 was end-labeled with $^{33}$P. The amplification mixtures

were resolved on an AFLP (sequence) gel, which was run, processed and exposed as described in Example 1.

The sequences of the adapters, AFLP preamplification primers and (selective AFLP) amplification primers used are as follows:

PstI adapter:

91D01: 5'-CTCGTAGACTGCGTACATGCA-3' (SEQ ID No.24 )

91D02 3'-CATCTGACGCATGT-5' (SEQ ID No.25 )

PstI +0 preamplification AFLP primer:

POOL: 5-GTAGACTGCGTACATGCAG-3' (SEQ ID No.26 )

PstI +2 selective amplification AFLP primer

P16: 5-GACTGCGTACATGCAGCC-3' (SEQ ID No.27 )

MseI adapter: (see example 1)

MseI+ 0 preamplification AFLP primer:

M00K (see example 2)

RAMP amplification primer:

98L39: 5'-GATAAGCGGAGAGAGA-3' (SEQ ID No.28)

3. Results

Figure 5 shows a section in the 180-330 basepairs fragment mobility size range of the fingerprint patterns obtained after separating the amplified fragments on a sequence gel. The parental lines B73 (P1) and A7(P2) and 32 F2 progeny as described in section 1 were loaded in the lanes indicated by P1, P2 and the numbers 1 through 32, respectively. The bands indicated by arrows may represent alleles of a polymorphic microsatellite repeat locus, targetted by the RAMP primer used.

4. Confirmation of targetting a microsatellite repeat by nucleotide sequencing

The two bands at the bottom part of Figure 5, amplified using primer combination P16/98L39 and indicated by arrows and a star (*) at the side, may represent two alleles of a microsatellite repeat locus. In order to demonstrate this, the nucleotide sequences of these fragments were determined after excision of the bands in B73 (P1) and A7 (P2) and re-amplification with the primers 98L39 and 98L89 according to the procedures described in Example 1.

98L89 (SEQ ID No.29):

5'-AGCGATAACAATTTCACACAGGATAGACTGCGTACCTGC-3'

The two resulting sequences, truncated at the PstI site, are given in SEQ ID NOs 30-31 and Figure 8, i.e.:

SEQ ID No.30: 170 basepairs: "upper" band (A7)
SEQ ID No.31: 168 basepairs: "lower" band (B73)

5. Conclusion

From Figure 5 and inspection of the sequences of SEQ IDs NOs 30-31 and Figure 8, it is apparent that the length difference between these fragments results from a 2 basepairs insertion/deletion polymorphism located at the boundary of a CT (GA) microsatellite-repeat. This microsatellite-repeat is targetted by RAMP primer 98L39. In addition, there are nine base substitutions distinghuising the two sequences. Thus, it is very likely that these two sequences are allelic sequences derived from the same microsatellite-repeat locus.

Example 4: Targetting of microsatellite repeat polymorphisms in pepper using the combination of an AFLP primer and a RAMP primer.

1. Description of biological materials

The biological materials used are the parental lines and 26 F2 progeny of pepper mapping population. The parental lines are Maor (indicated by P1 in Figure 6) and Perennial (indicated by P2 in Figure 6). The F2 progeny used are numbered 019-030, 032-034, 036-041,and 043-047.

2. EcoRI/MseI AFLP template preparation, preamplification and amplification using a selective EcoRI primer in combination with a RAMP primer.

AFLP templates prepared using the restriction enzymes EcoRI and MseI and preamplification reactions were carried out as described in Example 1 or references incorporated therein. Microsatellite-repeat fragment-enriched fingerprints patterns were obtained by carrying out a selective amplification from 20-fold diluted +0/+0 preamplification mixture using the combination of a EcoRI AFLP selective +2 AFLP primer and a RAMP primer. The amplification reactions were carried out as described in Example 1. For the results shown in Figure 6, EcoRI + 2 primers E15 was used in combination with RAMP primer 99A52, which is directed at CA-repeat sequences. RAMP primer 99A52 was end-labeled with $^{33}P$. The amplification mixtures were resolved on an AFLP (sequence) gel, which was run, processed and exposed as described in Example 1.

The sequences of the adapters, AFLP preamplification primers and (selective AFLP) amplification primers used are as follows:

EcoRI adapter: (see example 1)

EcoRI AFLP +0 preamplification primer: (see example 1)

EcoRI AFLP +2 selective amplification primer:

E15: 5'-GACTGCGTACCAATTCCA-3' (SEQ ID No.32 )

MseI adapter: (see example 1)

MseI AFLP +0 preamplification primer:

M00K (see example 2)

RAMP amplification primer:

99A52: 5'- GATAAGCGCACACACA-3' (SEQ ID No.33)

3. Results

Figure 6 shows a section in the 105-110 basepairs fragment mobility size range of the fingerprint patterns, obtained after separating the amplified fragments on a sequence gel. The parental lines Maor (P1), Perennial (P2) and 26 F2 progeny as described in section 1 were loaded in the lanes indicated by P1, P2 and the numbers 1 through 26, respectively. The bands indicated by arrows may represent alleles of a polymorphic microsatellite repeat locus, targetted by RAMP primer 99A52.

4. Confirmation of targetting microsatellite repeats by nucleotide sequencing

The two strong bands shown in Figure 6, which are indicated by arrows and a star (*) at the side, may represent two alleles of a microsatellite repeat locus. In order to demonstrate this, the nucleotide sequences of these fragments were determined after excision of the bands in Perennial (P2) and Maor (P2) and re-amplification with the primers 98A28 (see Example 2) and 99A52 according to the procedures described in Example 1.

The two resulting sequences, truncated at the EcoRI site, are given in SEQ ID Nos 34-35 and in Figure 8, i.e.:

SEQ ID No.34: 98 basepairs: "upper" band (Perennial)
SEQ ID No.35: 95 basepairs: "lower" band (Maor)

5. Conclusion

From Figure 6 and inspection of the sequences of SEQ ID Nos. 34 and 35 and Figure 8, it is apparent that the length difference between these fragments results from a 3 basepairs insertion/ deletion polymorphism located at the boundary of a GT (CA) microsatellite-repeat. This microsatellite-repeat is targetted by RAMP primer 99A52. In addition, there is a one base substitution in the internal sequence of the fragment distinguishing the two sequences. Thus, it is very likely that these two sequences are allelic sequences derived from the same microsatellite-repeat locus.

*Example 5: Partial complementary of fingerprint patterns generated using an AFLP primer in* combination with RAMP primers containing different anchor sequences at the 5'-prime end but identical dinucleotide repeat motifs.

1. Description of biological materials

The biological materials used are the parental lines and 6 F2 progeny of a maize mapping population. The parental lines are B73 (indicated by P1 in Figure 7) and A7 (indicated by P2 in Figure 7). The F2 progeny are numbered 021, 024, 025, 026, 027, and 031.

2. PstI/MseI template preparation and amplification using a selective AFLP primer in combination with RAMP primers with identical repeat sequences but different 5'-anchor sequences.

AFLP templates prepared using the restriction enzymes PstI and MseI and preamplification reactions were carried out as described in Example 1 or references incorporated therein. Microsatellite-repeat fragment-enriched fingerprints patterns were obtained by carrying out a selective amplification from 20-fold diluted +0/+0 preamplification mixture using the combination of a PstI AFLP selective +2 primer and a RAMP primer. The amplification reactions were carried out as described in Example 1. For the results shown in Figure 7, three RAMP primers with different 5 prime "anchor" sequences but identical dinucleotide repeat sequences were used. These RAMP primers are named 98L36, 98L37 and 98L76 and are directed a CT-repeat sequences. The amplification conditions used were as described in Examplel or references incorporated therein. For the fingerprint patterns shown in Figure 7, the P17 primer was end-labeled with $^{33}P$ and the amplification mixtures were resolved on a standard AFLP (sequence) gel, which was run, processes and exposed as described in Example 1.

The sequences of the adapters, AFLP- and RAMP primers used are as follows:

PstI +0 preamplification AFLP primer: (see example 3)

PstI +2 selective amplification AFLP primer

P17: 5-GACTGCGTACATGCAGCG-3' (SEQ ID No.36 )

MseI adapter: (see example 1)

MseI+ 0 preamplification AFLP primer:

M00K (see example 2)

RAMP amplification primer:

98L36: 5'-CAGCTAAGCTCTCTCT-3' (SEQ ID No.37 )

98L37: 5'-CAGCATGACTCTCTCT-3' (SEQ ID No.38 )

98L76: 5'-GATAAGCGCTCTCTCT-3' (SEQ ID No.39 )

3. Results

Figure 7 shows a section in the 250-500 basepairs fragment mobility size range of the fingerprint patterns obtained after separating the amplified fragments on a sequence gel. The fingerprint patterns obtained with three primer combinations are shown for the parental lines B73 (P1) and A7 (P2) and six F2 progeny (indicated by the lane number 1 through 6). The RAMP primers used differ only at the 5 prime anchor sequences and are 98L36 (indicated by anchor 1), 98L37 (indicated by anchor 2) and 98L76 (indicated by anchor 3).

Fragments pointed at by arrows connected to a solid line represent bands that are amplified by only one of the three RAMP primers, whereas fragments pointed at by arrows connected to a dashed line are amplified by more than one AFLP-RAMP primer combination.

4. Conclusion

Figure 7 shows that partially complementary fingerprint patterns are obtained when RAMP primers with different 5 prime anchor sequences but with identical microsatellite repeat sequences are used in combination with the same AFLP primer. This results suggests that amplification using RAMP primers with different anchor sequences is useful to maximize the number of targetted microsatellites.

Figure 7 further shows that amplification of fragments from AFLP preamplification reactions using the combination of an AFLP primer and a RAMP primer is based in part on mispriming between the RAMP anchor sequence on target sequences present in the AFLP preamplificaton mixture. With respect to the targetting of microsatellite repeats polymorphisms this does not have an adverse effect, as long as correct anchoring at the boundary of the repeats take place, as shown in Examples 1-4.

## Claims

1. Use of (the combination of) at least one RAMP-primer and at least one AFLP-primer in analysing a nucleic acid sequence, in particular in analysing a nucleic acid sequence for (the presence or absence of) polymorphisms/ markers associated with microsatellites.

2. Use of (the combination of) at least one RAMP-primer and at least one AFLP-primer in amplifying a nucleic acid sequence.

3. Use according to claim 2, in which said nucleic acid sequence comprises a restriction fragment to which at least one adapter sequence, and preferably two adapter sequences, has/have been ligated.

4. Use according to claim 3, in which said restriction fragment to which said at at least one adapter sequence has been ligated is part of a mixture of adapter-ligated restriction fragments.

5. Use according to any of claims 1-4, in which said nucleic acid sequence, and in particular said restriction fragment, contains or is suspected to contain, a microsatellite.

6. Use according to any of the preceding claims, in which said nucleic acid/restriction fragment is derived from genomic DNA, cDNA; and in particular eukaryotic genomic DNA, cDNA or (a mixture or a library of) recombinant DNA clones, e.g. derived from a plant, animal or a human.

7. Use according to any of the preceding claims, in which said nucleic acid/restriction fragment is derived from agronomically important crops such as wheat, barley, maize, tomato, pepper, lettuce, rice or soybean; from animals such as such as mouse, rat, pig, chicken or fish; or from a human being.

8. Method for analysing a nucleic acid sequence, said method at least comprising the steps of:

   (a) amplifying at least one restriction fragment generated from the nucleic acid to be analysed, in which said restriction fragment has been ligated to at least one adapter, with at least one RAMP-primer and at least one AFLP-primer to provide at least one amplified nucleic acid sequence;
   and optionally comprising the further step of:
   (b) detecting at least one of the amplified nucleic acid sequences thus obtained.

**9.** Method for analysing a nucleic acid sequence, said method comprising the steps of:

(a) restricting the starting nucleic acid with at least one restriction endonuclease to provide a mixture of restriction fragments;
(b) ligating the restriction fragments thus obtained to at least one adapter;
(c) amplifying the mixture of adapter-ligated restriction fragments thus obtained with at least one RAMP-primer and at least one AFLP-primer to provide a mixture of amplified restriction fragments;
and optionally comprising the further step of:
(d) detecting at least one of the amplified restriction fragments thus obtained.

**10.** Method according to claim 8 or 9, in which the starting nucleic acid is a nucleic acid that contains (or is at least suspected to contain) a microsatellite.

**11.** Method according to any of claims 8-10, in which the (starting) nucleic acid is a DNA sequence, more preferably a double stranded DNA sequence, such as genomic DNA, cDNA; and in particular eukaryotic genomic DNA, cDNA or (a mixture or a library of) recombinant DNA clones, e.g. derived from a plant, animal or a human.

**12.** Method according to any of claims 8-11, in which the starting nucleic acid is derived from agronomically important crops such as wheat, barley, maize, tomato, pepper, lettuce, rice or soybean; from animals such as such as mouse, rat, pig, chicken or fish; or from a human being.

**13.** Use of a RAMP-primer in the method of any of claims 8-12.

**14.** Use of an AFLP-primer in the method of any of claims 8-12.

**15.** Use according to any of claims 1-7 or 13-14, or method according to any of claims 8-12, in which the nucleotides of the RAMP-primer that form the anchor region are non-degenerate nucleotides, and preferably arbitrary non-degenerate nucleotides.

**16.** Use according to any of claims 1-7 or 13-15, or method according to any of claims 8-12 or 15, in which the RAMP primer contains a total of between 8 and 20 nucleotides, preferably between 12 and 16 nucleotides.

**17.** Use according to any of claims 1-7 or 13-16, or method according to any of claims 8-12 or 15-16, in which, of the nucleotides present in the RAMP-primer, between 6 and 8 nucleotides are (intended to be) complementary to (the repeat motive of) the microsatellite, the remainder of the nucleotides present in the RAMP-primer forming the anchor region.

**18.** Kit, comprising a RAMP-primer and an AFLP-primer; and optionally further containing an adapter complementary to said AFLP-primer and/or one or more components known per se for AFLP kits.

**19.** Data generated by the method of any of claims 8-12 or 15-17, optionally on a suitable data carrier, such as paper or a computer disk.

Fig 1

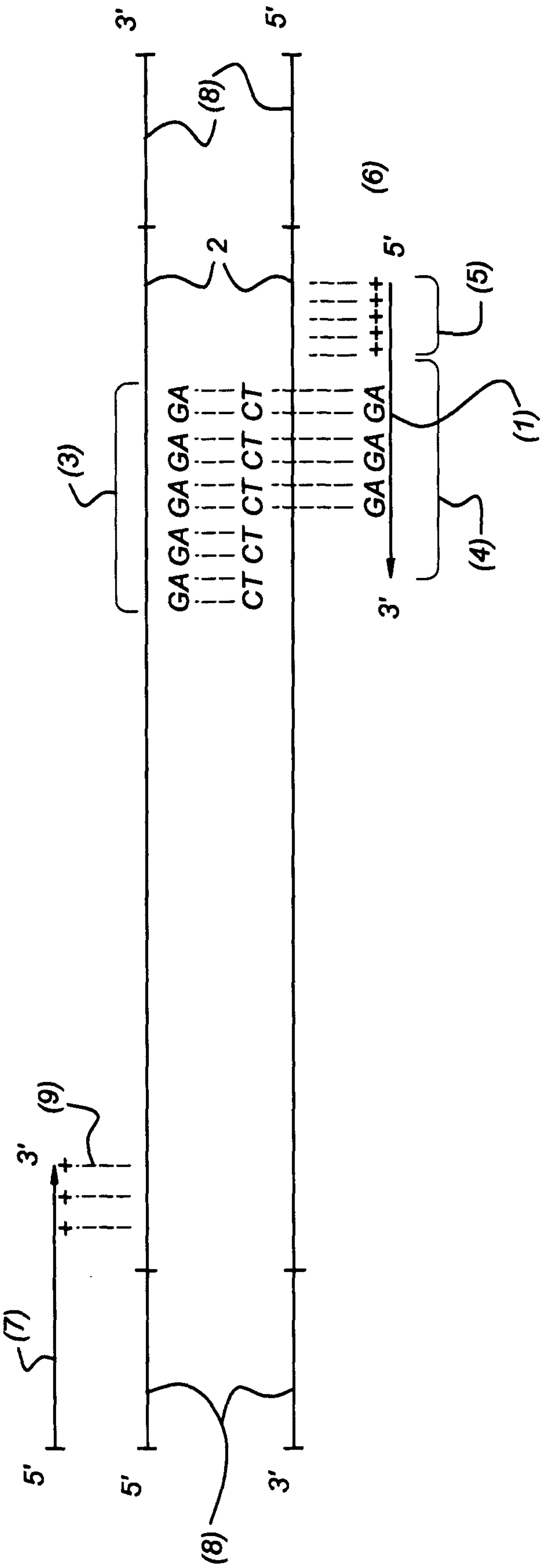

# Fig 2

1 Sequencing RAMP-fragment

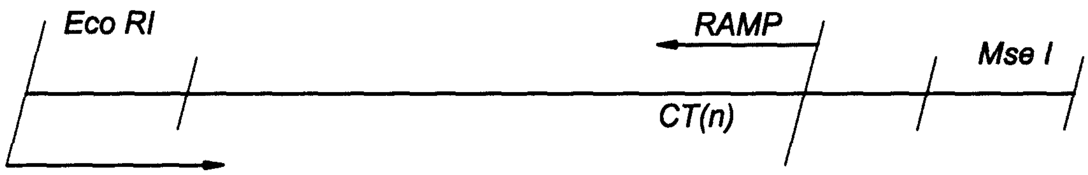


2 Amplification using internal PCR-primer-1 and Mse I-primer

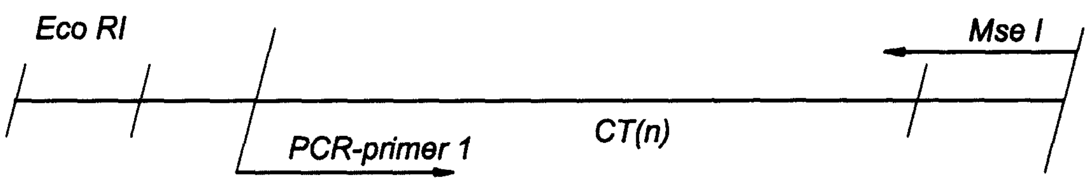


3 Sequencing amplified product PCR-primer-1 and Mse I-primer

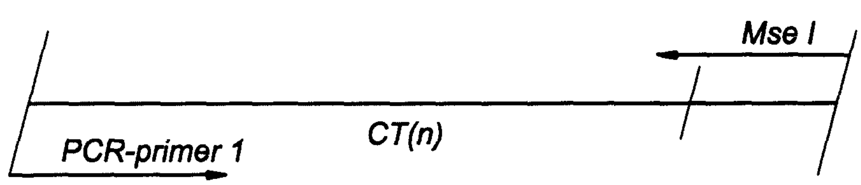


4 Amplification using PCR-primers 1 and 2

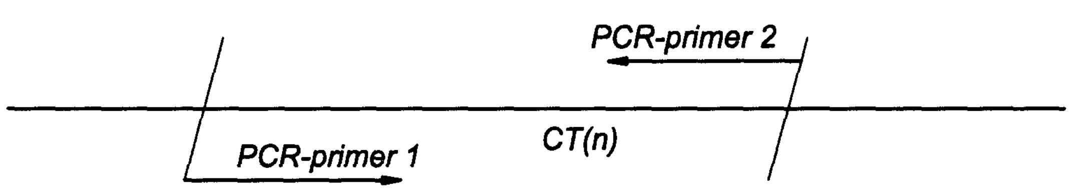

Fig 3

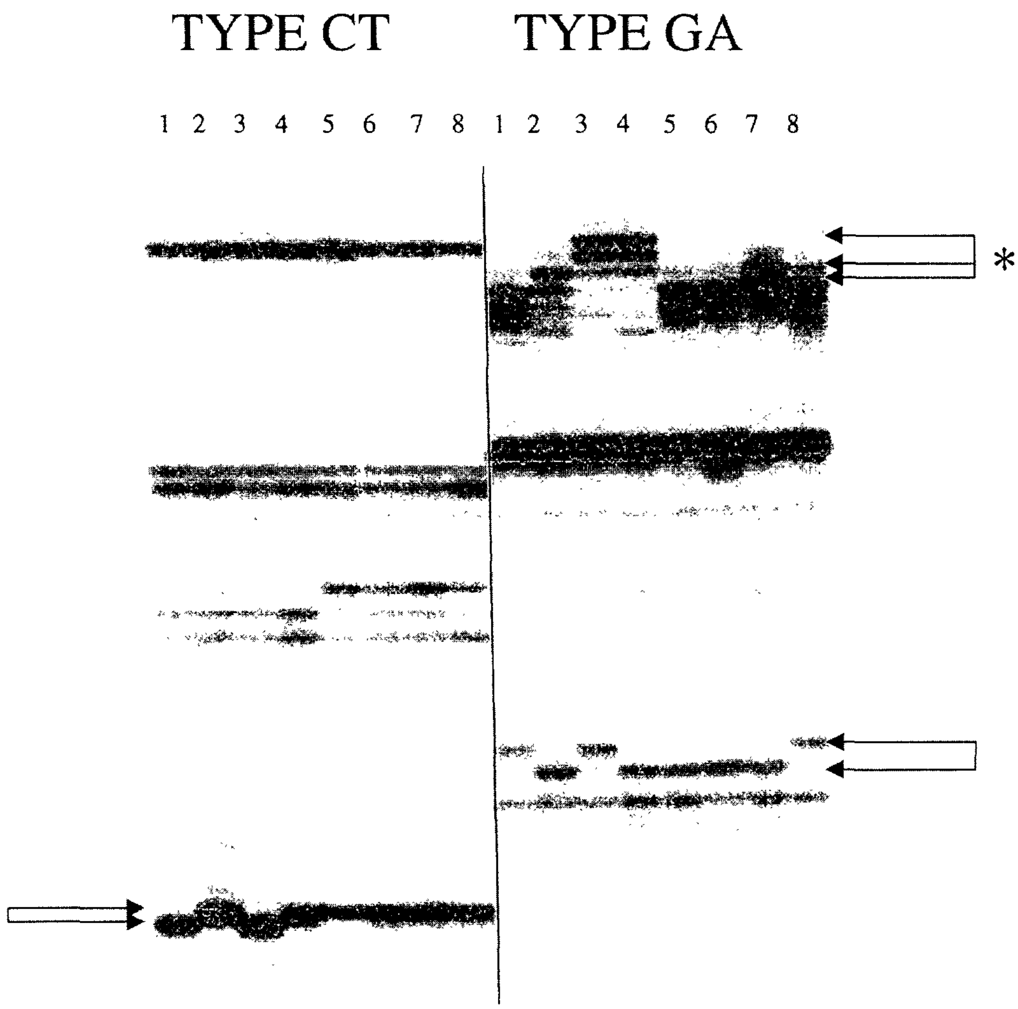

*Fig 4*

TYPE CA

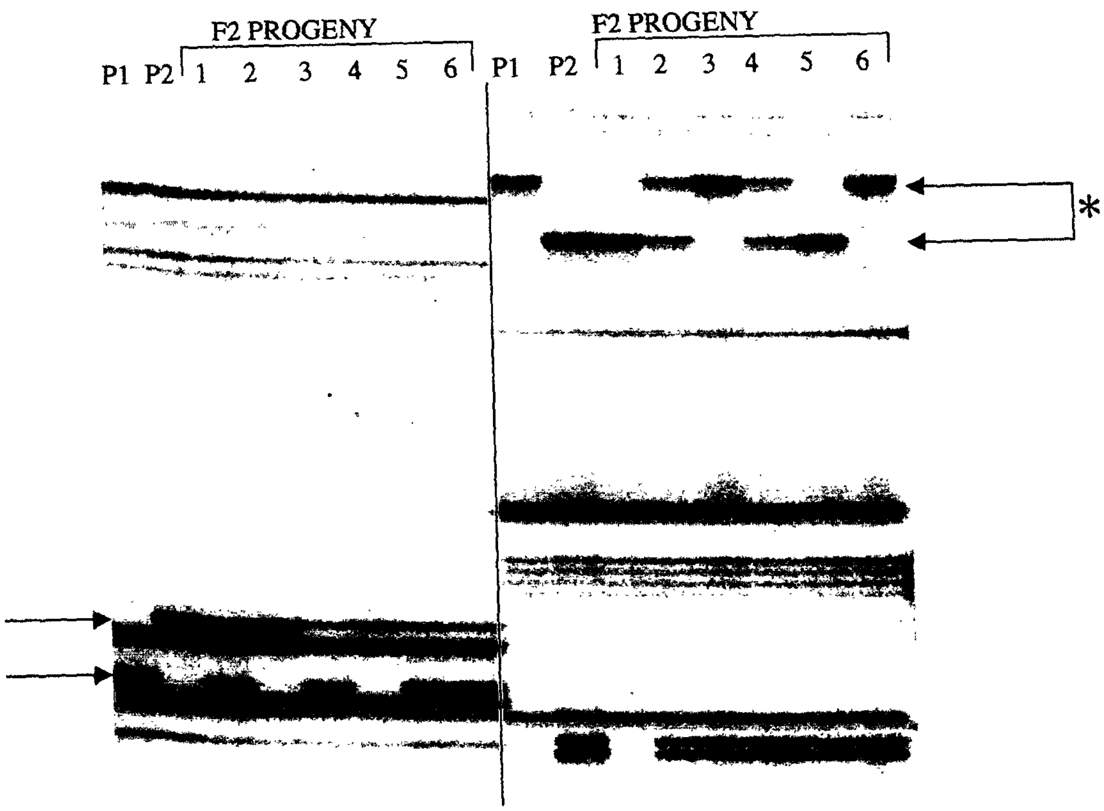

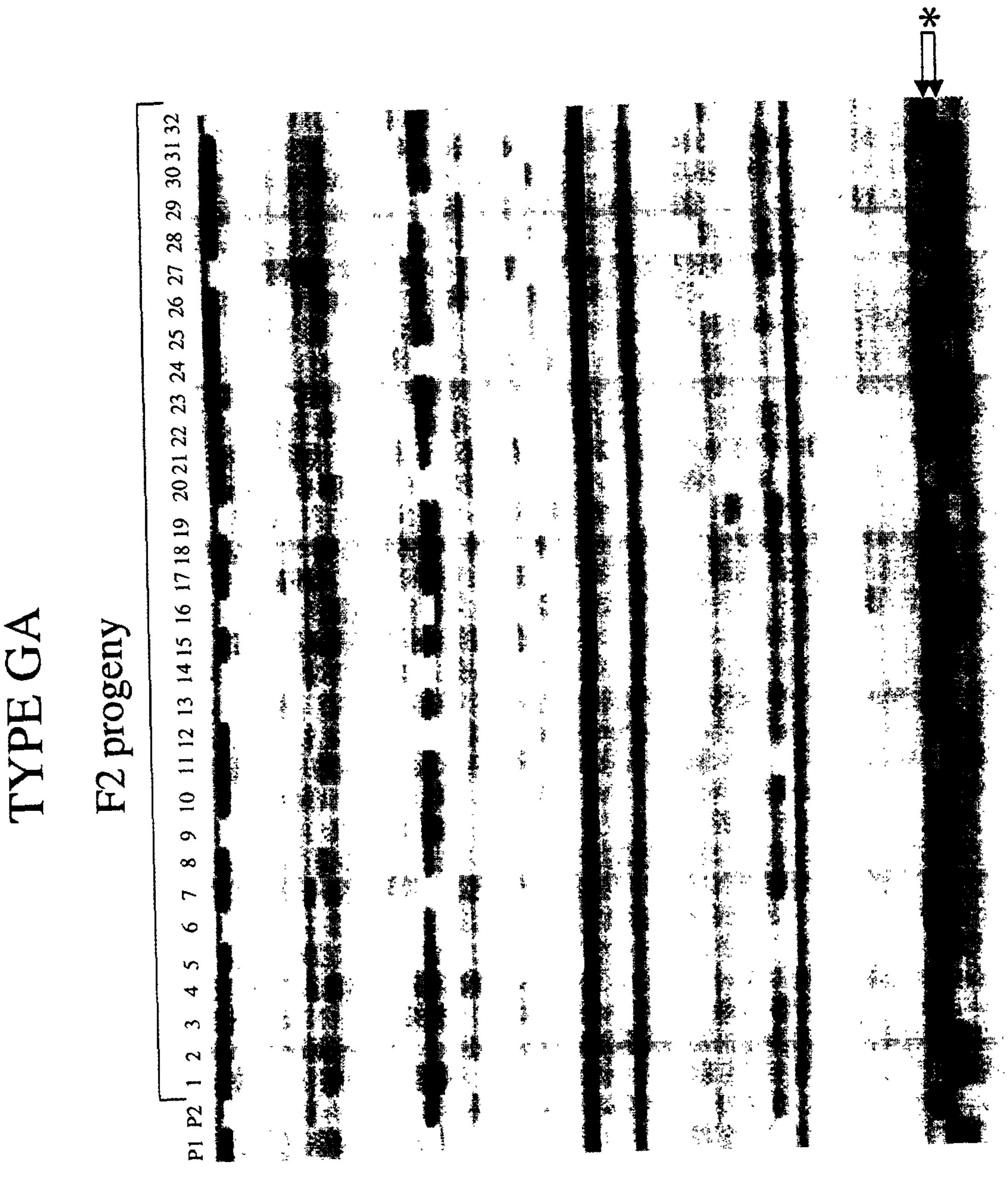
TYPE GA
F2 progeny
P1 P2 1 2 3 4 5 6 7 8 9 10 11 12 13 14 15 16 17 18 19 20 21 22 23 24 25 26 27 28 29 30 31 32
*

Fig 5

*Fig 6*

TYPE CA

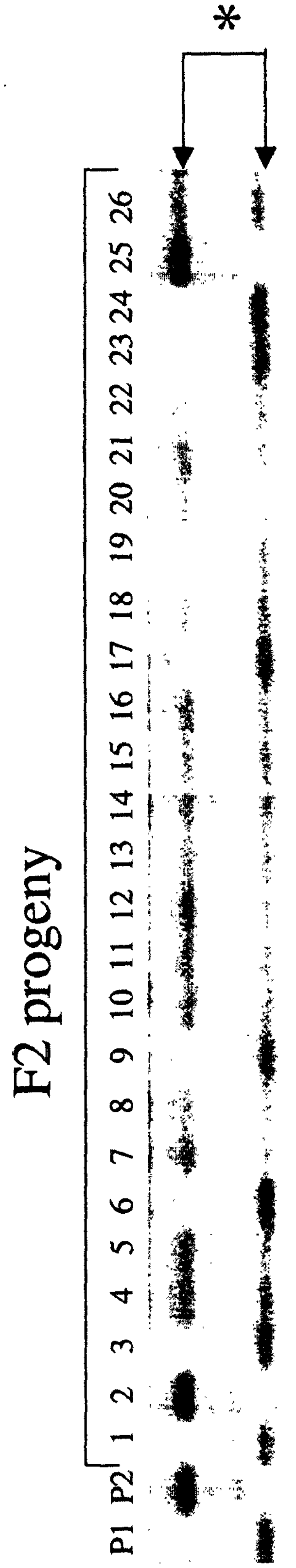
F2 progeny
P1 P2 1 2 3 4 5 6 7 8 9 10 11 12 13 14 15 16 17 18 19 20 21 22 23 24 25 26
*

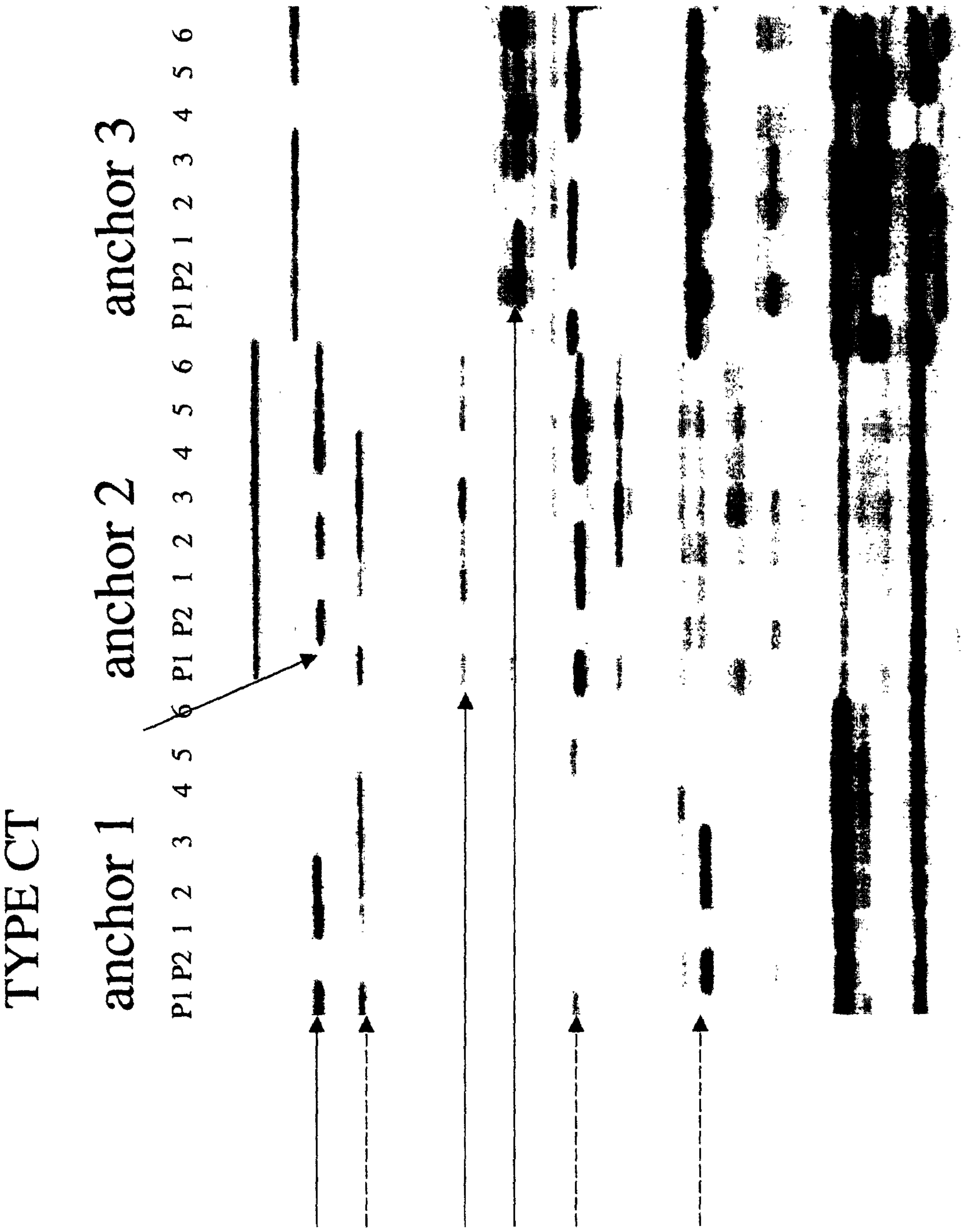
TYPE CT
anchor 1
P1 P2 1 2 3 4 5 6
anchor 2
P1 P2 1 2 3 4 5 6
anchor 3
P1 P2 1 2 3 4 5 6

Fig 7

# Fig 8a

A. Nucleotide sequences of lettuce fragments (the numbers refer to the SEQ IDs)

```
11.TCGACACAAAATCCGAGGCTGTCAGGCCCCCCACGACGTGGTGAAGAAGCCACGACGTGGTATTGTGATTGATGGGT
12.------------------------------------------------------------------------------
13.------------------------------------------------------------------------------

11.GCCAGGTGCGTCACGAAACGTCTCGTCAACAGAAGAGGANTAGAATCCCTCACCACGACGTGGTGACATGGCGCCCA
12.------------------------------------------------------------------------------
13.------------------------------------------------------------------------------

11.AATTAGGGCTATAAATAGCAGCCGAAGGTGTTGGGTTCCATTGCTTATTTCTTCTCTCTCTCTCTCTCTCTCTCTCT
12.----------------------------------------------------XXXX----------------------
13.----------------------------------------------------XXXXXX--------------------

11.CTCTCTCTCTCTCTCTCTCTCGTGCCGTC (260 bp)
12.----------------------------- (256 bp)
13.----------------------------- (254 bp)
```

# Fig 8b

B. Nucleotide sequences of pickle fragments (the numbers refer to the SEQ IDs)

```
22.GAATTCCGGGGCGARAACCAGAAGAAATAGAGAGGTATTGGATAATGACTCACCTTGAAGGGTTTGGAAAAAGAAGA
23.------------------------------------------------------------------------------

22.AGAGGATGAGAGAGACAGAGAGATACAGGCAATGAGGGTTCAGKCAATAAGTCCTCAAAGACTTCTGATGGGTTCTC
23.------------------------------------------------------------------------------

22.TACTTCATCGTTTGTGGTCTCCCTGAAGATAACAATCTCCCTAATAGCTTTAGTGTATTACAAGTACCCTTTCTAAG
23.----------------------XXXXXXXXXX----------------------------------------------

22.AACACATGGTATGGAACAGAGCTATCTTTTATCAATCAAATAACACAGTATAATTTATGATTCCAAATCAAAAACTA
23.------------------------------------------------------------------------------

22.AAGCAAAGAGCAAAGACTATTCAGTTCTTTTGTGTGTGTGTGTGTGTGTGTGTAAATCGAG (373 bp)
23.-----------------------------XXXXXX------------------------------ (357 bp)
```

# Fig 8c

C. Nucleotide sequences of maize fragments (the numbers refer to the SEQ IDs)

```
30 CTGCAGCCCTGCCGACCAGTCTCCGTTCCCCTTCTTGTATTTCATGGTGAGTAATAAGATTCTCTCTCTCTCTCT
31.----------A--------------------------------------A---------------------T-TG

30.TGTTTTTTCTGTCGAAAAATGGGAATCGCTACAAGTAGCATCCGTATTTTCCATTTTTTCCCGATACGATTCTCTCT
31.GT--------A---------------T-----------------------------------------XX-----

30.TCTCTCTCCGCTTATC (170 bp)
31.---------------- (168 bp)
```

# Fig 8d

D. Nucleotide sequences of pepper fragments (the numbers refer to the SEQ IDs)

```
34.GAATTCCATAAGGAGAGCTCGAATNAATGATCCATTTTAGTTGAAAGTTGAAATACAGTGCATCCTCTAATAGGAG
35.----------------------------------------------A----------------------------GG

34.TGTGTGTGTGTGCGCTTATC (98 bp)
35.GXXX---------------- (95 bp)
```

European Patent Office

# EUROPEAN SEARCH REPORT

Application Number
EP 00 20 1725

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 5 955 276 A (MORGANTE MICHELE ET AL) 21 September 1999 (1999-09-21)<br>* column 1-6; claims; figure 10; example 1 *<br>* column 19 *<br>* column 33, line 49-56 *<br>* column 34 *<br>* column 7, paragraph 2; figures 1,2 *<br>* column 9-16 *<br>--- | 1-19 | C12Q1/68 |
| X | WITSENBOER H ET AL: "Identification, genetic localization, and allelic diversity of selectively amplified microsatellite polymorphic loci in lettuce and wild relatives (Lactuca spp.)." GENOME,<br>vol. 40, no. 6, December 1997 (1997-12), pages 923-936, XP000946962<br>ISSN: 0831-2796<br>* page 923-927 *<br>--- | 1-19 | |
| X | WO 98 42867 A (FIRTH GREG)<br>1 October 1998 (1998-10-01)<br>* page 11; claim 1 *<br>* page 18-20 *<br>--- | 1-14, 16-18 | TECHNICAL FIELDS SEARCHED (Int.Cl.7)<br>C12Q |
| A,D | WU ET AL: "Detection of microsatellite polymorphisms without cloning"<br>NUCLEIC ACIDS RESEARCH,<br>vol. 22, no. 15, 1994, pages 3257-3258, XP002147007<br>* the whole document *<br>---<br>-/-- | 1-19 | |

The present search report has been drawn up for all claims

| Place of search | Date of completion of the search | Examiner |
|---|---|---|
| THE HAGUE | 17 October 2000 | Reuter, U |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 00 20 1725

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | SANCHEZ DE LA HOZ M P ET AL: "Simple sequence repeat primers used in polymerase chain reaction amplifications to study genetic diversity in barley." GENOME, vol. 39, no. 1, 1996, pages 112-117, XP000946960 ISSN: 0831-2796 * the whole document * --- | 1-19 | |
| A,D | VOS P ET AL: "AFLP: a new technique for DNA fingerprinting" NUCLEIC ACIDS RESEARCH, 11 November 1995 (1995-11-11), XP002086756 * the whole document * --- | 1-19 | |
| A | EP 0 721 987 A (KEYGENE NV) 17 July 1996 (1996-07-17) * the whole document * ----- | 1-19 | TECHNICAL FIELDS SEARCHED (Int.Cl.7) |

The present search report has been drawn up for all claims

| Place of search | Date of completion of the search | Examiner |
|---|---|---|
| THE HAGUE | 17 October 2000 | Reuter, U |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 00 20 1725

This annex lists the patent family members relating to the patent documents cited in the above-mentioned European search report.
The members are as contained in the European Patent Office EDP file on
The European Patent Office is in no way liable for these particulars which are merely given for the purpose of information.

17-10-2000

| Patent document cited in search report | Publication date | Patent family member(s) | Publication date |
|---|---|---|---|
| US 5955276 A | 21-09-1999 | AU 704660 B | 29-04-1999 |
| | | AU 4367496 A | 19-06-1996 |
| | | DE 69507646 D | 11-03-1999 |
| | | DE 69507646 T | 16-09-1999 |
| | | EP 0804618 A | 05-11-1997 |
| | | JP 10509594 T | 22-09-1998 |
| | | NZ 298236 A | 28-01-1999 |
| | | WO 9617082 A | 06-06-1996 |
| WO 9842867 A | 01-10-1998 | AU 6737698 A | 20-10-1998 |
| | | EP 0970246 A | 12-01-2000 |
| | | GB 2338553 A | 22-12-1999 |
| | | NO 994441 A | 22-11-1999 |
| | | CN 1257551 T | 21-06-2000 |
| EP 0721987 A | 17-07-1996 | AU 716571 B | 02-03-2000 |
| | | AU 4536196 A | 07-08-1996 |
| | | CA 2210410 A | 25-07-1996 |
| | | WO 9622388 A | 25-07-1996 |
| | | EP 0805875 A | 12-11-1997 |
| | | JP 11502406 T | 02-03-1999 |
| | | US 5874215 A | 23-02-1999 |

EPO FORM P0459

For more details about this annex : see Official Journal of the European Patent Office, No. 12/82

SEQUENCE LISTING

<110> Keygene N.V.

<120> Microsatellite-AFLP

<130> BO 43224

<140> EP
<141> 2000-05-15

<160> 39

<170> PatentIn Ver. 2.1

<210> 1
<211> 16
<212> DNA
<213> Artificial Sequence

<220>
<223> Description of Artificial Sequence: adapter

<400> 1
gacgatgagt cctgag 16

<210> 2
<211> 14
<212> DNA
<213> Artificial Sequence

<220>
<223> Description of Artificial Sequence: adapter

<400> 2
tactcaggac tcat 14

<210> 3
<211> 19
<212> DNA
<213> Artificial Sequence

<220>
<223> Description of Artificial Sequence:primer

<400> 3
gacgatgagt cctgagtaa 19

<210> 4
<211> 16
<212> DNA
<213> Artificial Sequence

<220>
<223> Description of Artificial Sequence:adapter

<400> 4
ctcgtagact gcgtac 16

<210> 5
<211> 13
<212> DNA
<213> Artificial Sequence

<220>
<223> Description of Artificial Sequence:adapter

<400> 5
cggtacgcag tct 13

<210> 6
<211> 16
<212> DNA

<213> Artificial Sequence

<220>
<223> Description of Artificial Sequence:primer

<400> 6
gtagactgcg taccga 16

<210> 7
<211> 19
<212> DNA
<213> Artificial Sequence

<220>
<223> Description of Artificial Sequence:primer

<400> 7
gtagactgcg taccgacac 19

<210> 8
<211> 16
<212> DNA
<213> Artificial Sequence

<220>
<223> Description of Artificial Sequence:primer

<400> 8
gataagcgct ctctct 16

<210> 9
<211> 16
<212> DNA
<213> Artificial Sequence

<220>

<223> Description of Artificial Sequence:primer

<400> 9
gacggcacga gagaga 16

<210> 10
<211> 49
<212> DNA
<213> Artificial Sequence

<220>
<223> Description of Artificial Sequence:primer

<400> 10
agcggataac aatttcacac aggacacact ggtatagact gcgtaccga 49

<210> 11
<211> 260
<212> DNA
<213> lettuce

<400> 11
tcgacacaaa atccgaggct gtcaggcccc ccacgacgtg gtgaagaagc cacgacgtgg 60
tattgtgatt gatgggtgcc aggtgcgtca cgaaacgtct cgtcaacaga agaggantag 120
aatccctcac cacgacgtgg tgacatggcg cccaaattag ggctataaat agcagccgaa 180
ggtgttgggt tccattgctt atttcttctc tctctctctc tctctctctc tctctctctc 240
tctctctctc tcgtgccgtc 260

<210> 12
<211> 256
<212> DNA
<213> lettuce

<400> 12
tcgacacaaa atccgaggct gtcaggcccc ccacgacgtg gtgaagaagc cacgacgtgg 60
tattgtgatt gatgggtgcc aggtgcgtca cgaaacgtct cgtcaacaga agaggantag 120

aatccctcac cacgacgtgg tgacatggcg cccaaattag ggctataaat agcagccgaa 180
ggtgttgggt tccattgctt atttcttctc tctctctctc tctctctctc tctctctctc 240
tctctctcgt gccgtc 256

<210> 13
<211> 254
<212> DNA
<213> lettuce

<400> 13
tcgacacaaa atccgaggct gtcaggcccc ccacgacgtg gtgaagaagc cacgacgtgg 60
tattgtgatt gatgggtgcc aggtgcgtca cgaaacgtct cgtcaacaga agaggantag 120
aatccctcac cacgacgtgg tgacatggcg cccaaattag ggctataaat agcagccgaa 180
ggtgttgggt tccattgctt atttcttctc tctctctctc tctctctctc tctctctctc 240
tctctcgtgc cgtc 254

<210> 14
<211> 17
<212> DNA
<213> Artificial Sequence

<220>
<223> Description of Artificial Sequence:adapter

<400> 14
ctcgtagact gcgtacc 17

<210> 15
<211> 15
<212> DNA
<213> Artificial Sequence

<220>
<223> Description of Artificial Sequence:adapter

<400> 15

aattggtacg cagtc 15

<210> 16
<211> 19
<212> DNA
<213> Artificial Sequence

<220>
<223> Description of Artificial Sequence:primer

<400> 16
gtagactgcg taccaattc 19

<210> 17
<211> 17
<212> DNA
<213> Artificial Sequence

<220>
<223> Description of Artificial Sequence:primer

<400> 17
gactgcgtac caattca 17

<210> 18
<211> 17
<212> DNA
<213> Artificial Sequence

<220>
<223> Description of Artificial Sequence:primer

<400> 18
gactgcgtac caattcc 17

<210> 19
<211> 16
<212> DNA
<213> Artificial Sequence

<220>
<223> Description of Artificial Sequence:primer

<400> 19
gatgagtcct gagtaa 16

<210> 20
<211> 16
<212> DNA
<213> Artificial Sequence

<220>
<223> Description of Artificial Sequence:primer

<400> 20
ctcgatttac acacac 16

<210> 21
<211> 40
<212> DNA
<213> Artificial Sequence

<220>
<223> Description of Artificial Sequence:primer

<400> 21
agcggataac aatttcacac aggatagact gcgtaccaat 40

<210> 22
<211> 373
<212> DNA

<213> pickle

<400> 22
gaattccggg gcgaraacca gaagaaatag agaggtattg gataatgact caccttgaag 60
ggtttggaaa aagaagaaga ggatgagaga gacagagaga tacaggcaat gagggttcag 120
kcaataagtc ctcaaagact tctgatgggt tctctacttc atcgtttgtg gtctccctga 180
agataacaat ctccctaata gctttagtgt attacaagta ccctttctaa gaacacatgg 240
tatggaacag agctatcttt tatcaatcaa ataacacagt ataatttatg attccaaatc 300
aaaaactaaa gcaaagagca aagactattc agttcttttg tgtgtgtgtg tgtgtgtgtg 360
tgtgtaaatc gag 373

<210> 23
<211> 357
<212> DNA
<213> pickle

<400> 23
gaattccggg gcgataacca gaagaaatag agaggtattg gataatgact caccttgaag 60
ggtttggaaa aagaagaaga ggatgagaga gacagagaga tacaggcaat gagggttcag 120
kcaataagtc ctcaaagact tctgatgggt tctctacttc atcgtttgtg gtctcccaat 180
ctccctaata gctttagtgt attacaagta ccctttctaa gaacacatgg tatggaacag 240
agctatcttt tatcaatcaa ataacacagt ataatttatg attccaaatc aaaaactaaa 300
gcaaagagca aagactattc agttcttgtg tgtgtgtgtg tgtgtgtgta aatcgag 357

<210> 24
<211> 21
<212> DNA
<213> Artificial Sequence

<220>
<223> Description of Artificial Sequence:adapter

<400> 24
ctcgtagact gcgtacatgc a 21

<210> 25

<211> 14
<212> DNA
<213> Artificial Sequence

<220>
<223> Description of Artificial Sequence:adapter

<400> 25
tgtacgcagt ctac 14

<210> 26
<211> 19
<212> DNA
<213> Artificial Sequence

<220>
<223> Description of Artificial Sequence:primer

<400> 26
gtagactgcg tacatgcag 19

<210> 27
<211> 18
<212> DNA
<213> Artificial Sequence

<220>
<223> Description of Artificial Sequence:primer

<400> 27
gactgcgtac atgcagcc 18

<210> 28
<211> 16
<212> DNA
<213> Artificial Sequence

<220>
<223> Description of Artificial Sequence:primer

<400> 28
gataagcgga gagaga 16

<210> 29
<211> 39
<212> DNA
<213> Artificial Sequence

<220>
<223> Description of Artificial Sequence:primer

<400> 29
agcgataaca atttcacaca ggatagactg cgtacctgc 39

<210> 30
<211> 170
<212> DNA
<213> maize

<400> 30
ctgcagccct gccgaccagt ctccgttccc cttcttgtat ttcatggtga gtaataagat 60
tctctctctc tctctcttgt tttttctgtc gaaaaatggg aatcgctaca agtagcatcc 120
gtattttcca ttttttcccg atacgattct ctcttctctc tccgcttatc 170

<210> 31
<211> 168
<212> DNA
<213> maize

<400> 31
ctgcagccct accgaccagt ctccgttccc cttcttgtat ttcatggtga gaaataagat 60
tctctctctc tcttttggtt tttttctatc gaaaaatggg aattgctaca agtagcatcc 120

gtattttcca ttttttcccg atacgattct cttctctctc cgcttatc 168

<210> 32
<211> 18
<212> DNA
<213> Artificial Sequence

<220>
<223> Description of Artificial Sequence:primer

<400> 32
gactgcgtac caattcca 18

<210> 33
<211> 16
<212> DNA
<213> Artificial Sequence

<220>
<223> Description of Artificial Sequence:primer

<400> 33
gataagcgca cacaca 16

<210> 34
<211> 98
<212> DNA
<213> pepper

<400> 34
gaattccata aggagagctc gaatnaatga tccattttag ttgaaagttg aaatacagtg 60
catcctctaa taggagtgtg tgtgtgtgtg cgcttatc 98

<210> 35
<211> 95

<212> DNA
<213> pepper

<400> 35
gaattccata aggagagctc gaatnaatga tccattttag ttgaaaattg aaatacagtg 60
catcctctaa taggggtgt gtgtgtgcgc ttatc 95

<210> 36
<211> 18
<212> DNA
<213> Artificial Sequence

<220>
<223> Description of Artificial Sequence:primer

<400> 36
gactgcgtac atgcagcg 18

<210> 37
<211> 16
<212> DNA
<213> Artificial Sequence

<220>
<223> Description of Artificial Sequence:primer

<400> 37
cagctaagct ctctct 16

<210> 38
<211> 16
<212> DNA
<213> Artificial Sequence

<220>
<223> Description of Artificial Sequence:primer

```
<400> 38
cagcatgact ctctct                                                          16


<210> 39
<211> 16
<212> DNA
<213> Artificial Sequence

<220>
<223> Description of Artificial Sequence:primer

<400> 39
gataagcgct ctctct                                                          16
```